# EUROPEAN PATENT APPLICATION

(11) **EP 4 591 816 A1**
(43) Date of publication of application: **30.07.2025**
(21) Application number: 24154551.6
(22) Date of filing: 29.01.2024
(51) Int. Cl.: A61B 18/22

(54) **HEATING PROBE**

(71) Applicant: Clinical Laserthermia Systems AB, 223 81 Lund (SE)
(72) Inventor: PANTALEONE, Cristina, 222 40 Lund (SE); KNAPPE, Verena, 214 65 Hamburg (DE); Dymling, Stephan, 216 22 Limhamn (SE)
(74) Representative: KIPA AB

(57) **Abstract**

A heating probe for performing thermotherapy on at least a portion of a tissue site. The probe includes an optical waveguide which comprising a light emitting area arranged at a distal portion, wherein the distal portion is partially a diffusor made from a structured writing and the structured writing comprising micro-modifications and the micro-modifications are burnt into a core and/or cladding and/or buffer of said optical waveguide. The distal portion has an optical axis and the micro-modifications are arranged along the optical axis so that light is emitted from both a lateral surface and an end surface.

## Description

### TECHNICAL FIELD

The present disclosure relates in general to the field of therapy of tissue. Especially, interstitial therapy using a heat probe which includes an optical waveguide having a diffuser at the tip for applying light to heat the surrounding tissue. More particularly the invention relates to an optical waveguide with a diffuser tip having a controlled forwards radiations for treatment sites where the mechanical penetration of the heat probe has to be shallower than normal.

### BACKGROUND

The treatment of tissues by electromagnetic radiation in the form of laser beams is known. Typically, a laser source produces a beam of laser light which is fed via a waveguide, for example an optical fibre, though the body of a surgical probe to a light-transmission probe tip from which the light can leave the surgical probe and heat up the tissue in the vicinity of the probe tip. This type of treatment requires a high degree of precision in correct positioning of the surgical probes in order to concentrate the heat transfer to an intended target area of the tumour. Incorrect positioning of the surgical probe will result in lesser treatment effect and may potentially damage surrounding tissue. In many applications, such as for example treatment of a prostate gland with a tumour, or a treatment site in brain tissue, the surrounding areas are sensitive to unintended damage which may result in serious negative ramifications for the patient.

To improve the heat treatment procedures, various diffusers has been developed with the aim of maximising the radial radiation of light and at the same time obtain a diffuser with good thermal and mechanical properties. Such diffuser made from micro-modifications is described in WO 2016/202328 A1 and in WO 2018/109139 A1.

However, in some treatment situations the optimal position requires the surgical probe to be inserted deeply into tissue where the tip of the probe is very close to critical structures with a large risk of mechanical damage inflicted by the surgical probe. This is a known problem that makes certain procedures risky or not possible to be conducted using laser-based treatment procedures.

It would therefore be an advantage for patients' safety, and to expand the number or procedures that would be possible to carry out, to have a heat probe which could be used in these circumstanced where the optimal position normally requires the surgical probe to be inserted deeply into tissue and close to critical structures. A further advantage would be if the complexity of the system used for the treatment can be reduced.

### SUMMARY

Accordingly, embodiments of the present invention preferably seek to mitigate, alleviate or eliminate one or more deficiencies, disadvantages or issues in the art, such as the above-identified, singly or in any combination by providing an improved heating probe for treatment of a tissue site. In particular treatment sites where the mechanical penetration of the heat probe has to be shallower than normal, which includes, for example, treatments sites in brain tissue as well as prostate.

According to a first aspect of the disclosure, a heating probe for performing thermotherapy on at least a portion of a tissue site is described. The heating probe may include an optical waveguide which comprising a light emitting area arranged at a distal portion. The distal portion may partially be a diffusor made from a structured writing. The structured writing may include micro-modifications and the micro-modifications may be burnt into a core and/or cladding and/or buffer of the optical waveguide. The distal portion may have an optical axis and the micro-modifications may be arranged along the optical axis so that light is emitted from both a lateral surface and an end surface.

In some examples, may the micro-modifications be arranged as a helix or spiral along the optical axis.

In some examples may the micro-modifications are arranged as at least two helices or spirals, wherein each spiral and helix has a different radii.

In some example may the helices and spirals be arranged with an offset in relation to each other in a direction of the optical axis.

In some examples may the number of helices and spirals varies along the optical axis.

In some examples may the micro-modifications be arranged as a plurality of lines arranged along the optical axis.

In some examples may the lines have different distances from the optical axis.

In some examples may the number of lines varies along the optical axis.

In some examples may the density of the micro-modifications varies along the optical axis to control the portion of light being emitted from the lateral surface and the end surface.

In some examples may the density increase towards the end surface.

In some examples may the density vary by varying the distance between the micro-modifications along the optical axis.

In some examples may a capillary be arranged to cover at least said light emitting area to protect the emitting area.

In some examples may the capillary be configured to lower energy density in an interface between the heating probe and the tissue.

In some examples may tissue be brain tissue for treatment of a neurological disease, such as brain tumour, radiation necrosis or epilepsy.

In a further aspect of the disclosure is a system for performing thermotherapy on at least a portion of a tissue site described. The system may include a light source configured to emit light and a heating probe configured to be inserted into the tissue site. The heating probe may include an optical waveguide which comprising a light emitting area arranged at a distal portion, wherein the distal portion is partially a diffusor made from a structured writing. The structured writing may include micro-modifications and the micro-modifications may be burnt into a core and/or cladding and/or buffer of the optical waveguide. The distal portion may have an optical axis and the micro-modifications may be arranged along the optical axis so that light are emitted from both a lateral surface and an end surface.

In some examples may magnetic resonance imaging (MRI) be used for monitoring the heat distribution in the tissue site and/or tissue surrounding the tissue site.

In some examples may at least one temperature probe be used for monitoring the heat distribution in the tissue site and/or tissue surrounding the tissue site.

It should be emphasized that the terms "comprises/comprising" and "exhibits, exhibiting" when used in this specification are used to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which:
Figure 1 shows a schematic setup of an optical waveguide with micro-modifications induced by laser radiation, according to one or more examples of the disclosure.
Figure 2 shows a schematic illustration of an optical waveguide and the input coupling of focussed laser light, and the possibility of relative movement between the focussed laser light and the optical waveguide, according to one or more examples of the disclosure.
Figure 3 shows a schematic setup of the processing device for processing optical waveguides a) in a frontal view and b) in a lateral view, according to one or more examples of the disclosure.
Figure 4 shows a method for processing optical waveguides using laser radiation, according to one or more examples of the disclosure.
Figures 5A and 5B show schematics of a diffusing waveguide end, according to one or more examples of the disclosure.
Figures 6A to 6H show schematics of arrangements of micro-modifications in spiral or helix arrangements, according to one or more examples of the disclosure.
Figures 7A to 7D show schematics of arrangements of micro-modifications in line arrangements, according to one or more examples of the disclosure.
Figures 8A to 8G show schematics of arrangements of micro-modifications in circular arrangements, according to one or more examples of the disclosure.
Figure 9 shows a schematic of capillary arranged to protect an emitting area of an optical waveguide, according to one or more examples of the disclosure.
Figures 10A to 10G show schematics with arrangement to protect an emitting area of an optical waveguide, according to one or more examples of the disclosure.
Figure 11 shows a schematic illustration of a treatment system.
Figures 12A and 12B show schematic illustrations of a treatment system.

### DETAILED DESCRIPTION OF EXAMPLES OF THE DISCLOSURE

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

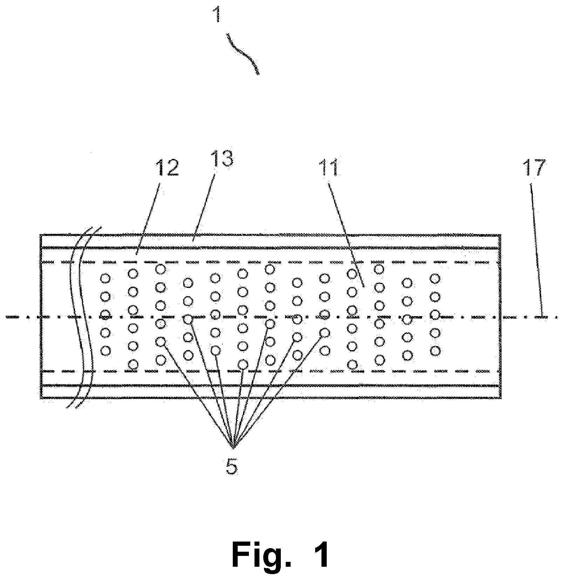

FIG. 1 shows a schematic illustration of the optical waveguides 1 to be machined. The optical waveguide comprises a first region 15 which is largely free from micro-modifications 5 and a second region 16 of the optical waveguide 1 into which micro-modifications 5 have been introduced. The micro-modifications may be a change of the optical properties of the material into which the micro-modification is written, such as a modification of refractive index, such as a modification of the refractive index of a core of the optical waveguide. The modification of the refractive index may include cracks in the core of the optical waveguide. The sizes of the micro-modifications could be between 10-15 µm. This region 16 is usually arranged at the distal portion of the optical waveguide 1. Arranging the second region 16 at a distal portion of the optical waveguide 1 may include a small distance between the distal end surface of the optical waveguide 1 and an end of the second region 16. The distance between the distal end surface of the optical waveguide 1 and an end of the second region 16 may be in the range of a few mm, such as 1 to 5mm, such as 1 to 3mm, such as 1 to 2mm such as 1mm. Optionally, the optical waveguide can be provided with a capillary, covering at least partially the section of the waveguide having micro-modifications 5.

In the figures, an optical waveguide 1 with a single core is illustrated. In some examples, the optical waveguide 1 may include multiple cores. A second region 16 which includes micro-modification 5 may then be written to at least one of the multiple cores, such as a plurality of the multiple cores, such as all of the multiple cores.

The core 11 is surrounded by cladding 12, cladding 12 may include multiple layers of cladding, followed by a coating and/or a buffer 13. The core 11 and cladding 12 usually consist of quartz and are doped differently. The refractive index of the cladding material is less than that of the core material; in this manner, the light can be transported in the optical waveguide 1 as a result of total internal reflection at the core-cladding transition. The cladding 12 is surrounded by a so-called coating and/or buffer 13, which takes up the tension when bending the optical waveguide 1 and therefore ensures the destruction-free resilience, and likewise serves for the protection against mechanical effects on the layers lying there below. Outside the coating and/or buffer 13 may a jacket be arranged. For the purposes of processing the optical waveguide 1, it is possible to remove the jacker, coating and/or buffer 13 that is not transparent to the selected laser wavelength such that the laser light only still needs to be focussed through the cladding 12. In the case of the jacket, coating and/or buffer 13 material that is transparent to the selected laser wavelength, e.g. nylon or PTFE, the optical waveguide 1 can also be processed through the jacket, coating and/or buffer 13. The same applies if there are multiple jackets or layers of coating and/or buffers 13 transparent to the selected wavelength. Not having to remove the jacket, coating and/or buffer 13 is advantageous in that the processed region of the optical waveguide 1 substantially has the same increased rigidity as the rest of the optical waveguide 1.

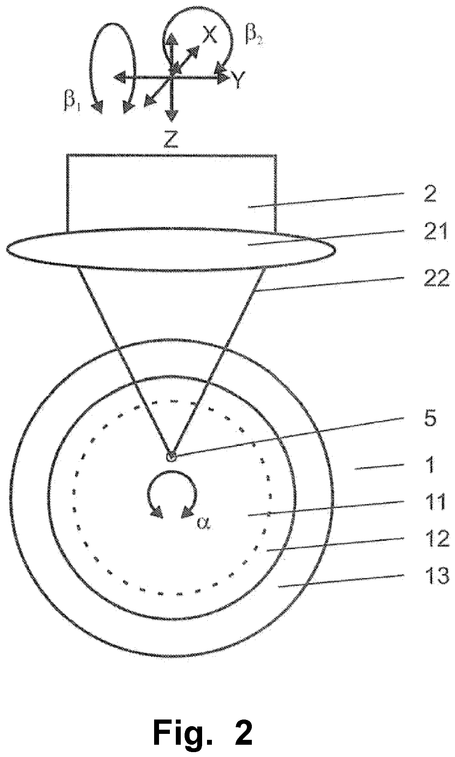

FIG. 2 similarly depicts the principle of coupling the focussed laser light 2 into the optical waveguide 1 for introducing the micro-modifications 5. FIG. 2 shows the focusing optical unit 21 required for focusing the laser pulses into the core region of the optical waveguide 1 and the generated micro-modifications 5. In this case, important lens parameters for introducing the micro-modifications 5 into the optical waveguide 1 are the focal length and the numerical aperture (NA) of the symbolically depicted focusing optical unit 21. The focal length is selected to be as short as possible as this allows the size of the focal points to be minimized. However, in so doing, the focal length needs to be long enough to be able to focus into the core 11 through the optical waveguide cladding. In a preferred variant, the focal length of the focusing optical unit 21 lies between 1 and 5 mm. However, the use of "long-distance" microscope lenses with the work distance of greater than 5 mm is also a preferred option of implementation. An NA of the focusing optical unit 21 which is as large as possible is also advantageous since this determines the aperture angle of the focusing optical unit 2. The greater the aperture angle is, the shorter the focal region. This is of great importance as it allows the extension in depth of the introduced modifications 5 to be minimized. The larger aperture angle leads to higher beam divergence and, as a result thereof, to a quickly increasing beam diameter upstream and downstream of the focal point. The same concept may be applied to the focal length. By controlling the focal length, such as using a short focal length, the extension in depth of the focal point and the size of the focal region may be controlled to shape the micro-modifications as a round dot or point, or to be shaped as a small cylinder. This reduces the energy density in the regions upstream and downstream of the focus and therefore also reduces the absorption and the risk of damage outside of the focal region.

In a particularly preferred variant, a short focal length (f<3.1 mm) aspherical lens with a numerical aperture NA>0.68 is used as a focusing optical unit 21. In a further example, use is made of a special lens (lens element system) with a high NA. It is constructed in such a way that the wavefronts of the focussed laser radiation 22 have the same radius of curvature as the material surface on which they are incident. This is advantageous in that the wavefronts are not distorted (wave front distortion) when passing through the optical waveguide surface, which in turn leads to a significantly improved focusability in the material of the optical waveguide 1.

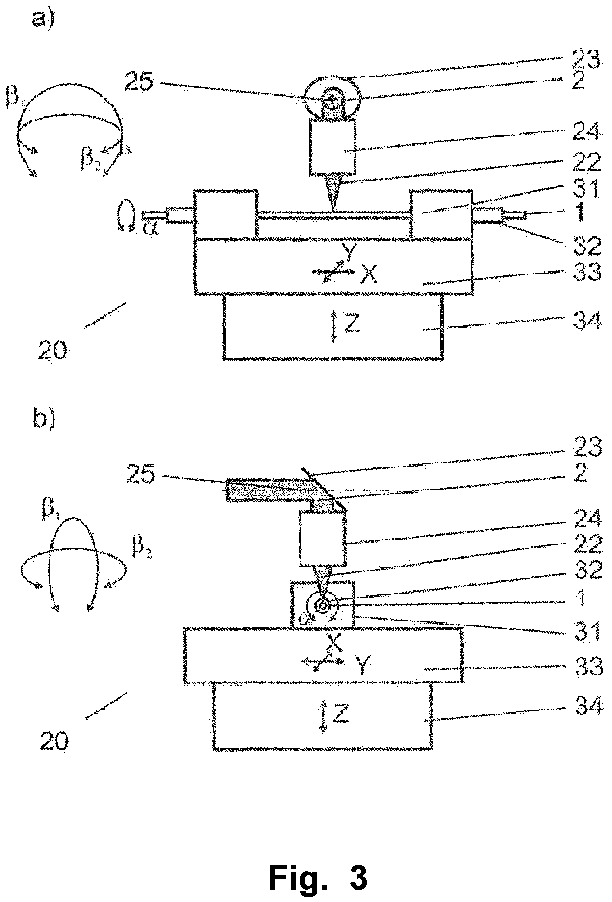

FIG. 3 shows a schematic diagram of the device according to the disclosure for introducing micro-modifications into optical waveguides 20. The device 20 comprises various motor-driven adjustment devices 33, 34 for carrying out a linear movement between the optical waveguide 1 and the focus of the focussed laser beam 22. The movement is preferably carried out in the spatial directions (X, Y, Z) by way of linear drives 33, 34. Furthermore, the device 20 comprises the setup for coupling 23 the laser light 2 into the focusing optical unit 24. Furthermore, the device 20 comprises the holder 32 for the optical waveguide 1 and the axes of rotation (α, β₁, β₂, β₃) for rotation of same. In contrast to the previously known solutions, it is not the focusing lens that is moved but only the optical waveguide 1. This is advantageous in that there is no need to displace or move deflection mirrors in the beam path during the processing. As a consequence, the set up or adjustment outlay for the device is significantly reduced and, at the same time, an improved long-term stability of the setup emerges since all optical elements can be securely installed in the beam path. By way of example, in the case of deflection mirrors moved by translation, even small inaccuracies or deviations in the beam path would lead to the laser beam 2 migrating on the focusing optical unit 24. As a result, the focal point migrates both in the XY-plane and in the Z-direction due to the beam passage through the focusing optical unit 24 which is at an angle relative (not perpendicular) to the optical waveguide 1. A setup with long-term stability and reproducible processing results can only be realized with much difficulty in this manner or not at all.

The Z-shaft 34 carries the further processing setup consisting of X- and Y-shaft 33, rotation device 31 and holder/guide 32 for the optical waveguides 1. It serves to move the optical waveguide 1 towards the focusing optical unit 24 or away from the latter. In this manner, it is possible to vary the distance between the focal point and the centre point of the optical waveguide 1, i.e. the depth position. The X-shaft 33 serves to displace the optical waveguide or the holder/guide 32 along the extent of the optical waveguide under the focusing optical unit 24. Thus, the maximum length of a modified region is only determined by the maximum travel of this shaft. The Y-shaft 33 moves the holder/guide 32 at right angles to the extent of the optical waveguide under the focusing optical unit 24. It serves to control the alignment of the micro-modifications 5 since the Y-shaft 33 can be used to align the focusing optical unit 24 and the optical waveguide 1 relative to one another in such a way that the laser beam 2 is incident as perpendicularly as possible on the optical waveguide surface. An oblique incidence on the surface leads to a modified beam path with a distortion of the focal region and therefore influences not only the alignment but also the form and size of the introduced modifications. The employed laser beam 20 is usually guided into the focusing optical unit 24 via a deflection mirror 23, although this is not mandatory. The optical waveguide 1 to be processed is held by a holder and guide 32 in an exact position in front of the focusing optical unit 24. This guide is cut out in the region of the processing or it is transparent to the employed laser radiation 2 and 22. The rotation device 31 serves to rotate the optical waveguide 1 about the longitudinal axis thereof. To this end, the optical waveguide 1 is fastened to the rotation device 31 by means of the tensioning device. In order to avoid excessive torsional tension of the optical waveguide 1, the latter is in this case always only rotated step-by-step by up to 360 degrees and subsequently rotated by up to 360 degrees in the opposite direction. This is realizable both for loose optical waveguide portions, e.g. finished optical waveguides, and for roll-to-roll production processes, in which the optical waveguides 1 can obtain any length. Alternatively, the waveguide 1 is fastened to the rotational device 31 which includes a motor system. The waveguide 1 is fastened to the rotational device 31 with a motor at each end of the waveguide 1. The two motors are then rotating the waveguide 1 synchronized multiple times, (i.e. more than 360 degrees) to avoid create tension. After the first structure, such as a spiral or helix, of micro-modifications has been obtained in the in the light diffusing region, the focal point is arranged at the initial start position of the first structure and then is a second structure, such as a spiral or helix, of micro-modifications obtained in the light diffusing region by rotating the optical waveguide 1 in the opposite direction. Alternatively, the waveguide may be rotated in the same direction as the first structure when obtaining the second structure of micro-modifications 5.

In other words, the device comprising a shaft and motor system is provided for the method for introducing micro-modifications into optical waveguides. Firstly, this device serves to hold the optical waveguide and, secondly, the device allows a targeted displacement and rotation of the optical waveguide and it enables arbitrary positioning of the focus of the laser system within the optical waveguide. The method according to the invention and the associated device according to the invention allow any variation of the form, the distribution and the position of the micro-modifications within the optical waveguide. As a result of this, it is possible, for example, to influence the position or the form of the micro-modifications in a targeted manner by way of the displacement speed of the linear and rotational axes or by varying the repetition rate. The distribution of the micromodifications is also controllable in a targeted manner, for example by way of a so-called laser-internal "pulse picking" or by way of a programmable shutter. The depth of the micro-modifications in the optical waveguide 1 may be influenced by a targeted movement of the focus or by a targeted adjustment of the focusing apparatus. Furthermore, an extension in depth of the individual micro-modifications can be influenced in a targeted manner by an appropriate selection of individual pulse energy, pulse duration, pulse number (individual pulse, double pulse, multiple pulse), spatial distance and/or time interval, or else by "pulse tailoring". It is likewise possible to introduce micro-modifications into the optical waveguide on the side facing away from the laser system by way of focusing through the middle of the optical waveguide. Here, it is possible additionally to use lens-like effects of the curved surface of the optical waveguide for the focusing in
order thus to generate micro-modifications which have an even shorter extension in depth. The arrangement of the micro-modifications is also influenced by the focal position or the positioning of the focusing apparatus relative to the optical waveguide axis. Thus, the alignment of the micromodification can be controlled by shifting the incoming beam position from the normal of the optical waveguide axis.

Any parameter which can be used to describe the micro-modifications, e.g. the depth position, the spatial extent, the distribution, the distance from one another, the position, the alignment or else the form of the micromodifications, can have an influence on the mechanical stability of the optical waveguide. The smaller the spatial extent of the micro-modifications and the greater the distance between the micro-modifications, the smaller the influence is on the mechanical stability of the optical waveguide. On the other hand, it is also the case that the strength of the light decoupling caused by the micro-modifications behaves in a substantially opposite manner to the effects on the mechanical stability. Therefore, it is essential to find a compromise between the decoupled light intensity and the mechanical or thermal stability of the optical waveguide. After a region of micro-modifications have been obtained, a thermo-camera may be used to check the quality by locating any potential hotspots in the diffusing waveguide tip.

Various embodiments are conceivable in relation to the distribution of the micro-modifications. By way of example, it is possible to produce a deliberate irregular distribution of the micro-modifications in order to avoid grating effects, such as e.g. interferences, and in order to simultaneously ensure a uniform distribution of the decoupled light. On the other hand, a targeted regular or periodic distribution of the micro-modifications, such as e.g. a Bragg grating or else multidimensional photonic structures, is also conceivable. Moreover, the option exists of placing the micro-modifications so tightly in the interior of the optical waveguide that these modifications form a line which itself has waveguide properties. This line structure can have any length and the extent thereof relative to the optical waveguide can likewise have any embodiment; thus, for example, a straight line or spiral or helix embodiment is conceivable.

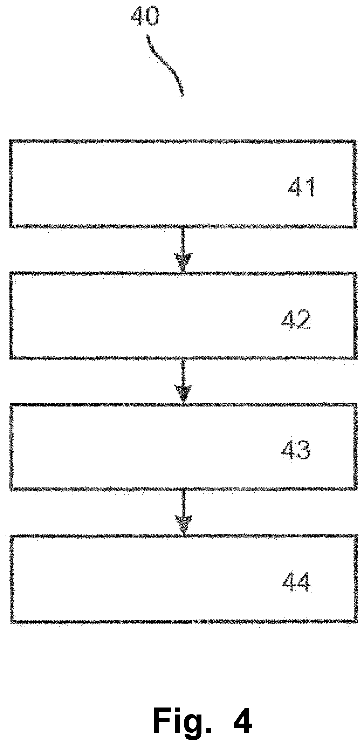

FIG. 4 depicts a method for processing optical waveguides 1 using laser radiation 2 in one example of the disclosure. Initially, the optical waveguide 1 is fixed in terms of its position with the aid of a holder/guide 32, 41. The holder/guide 32 is designed in such a way that the region of the optical waveguide (1) in which the micromodifications are intended to be generated is accessible for the laser radiation 2. The optical waveguide is mounted in such a way that it is movable in three spatial directions in relation to the focal position. This can be achieved by a movable optical unit 24 and a rigid mount of the optical waveguide (1) or by a rigid optical unit 24 and a movably arranged optical waveguide (1). The movement options comprise the three spatial directions X, Y and Z and the rotation α about the longitudinal axis of the optical waveguide 1 and/or the rotation β₁, β₂, β₃ about one or more axes. The laser beam 2 is focussed in a further method step 42. The focussed laser beam 22 is positioned in such a way that, with the aid of the movement options, the position of the focus is movable through the whole region in which the micro-modifications are intended to be introduced. The focal position is moved by the optical waveguide according to a predetermined pattern 43. Preferably, use is made of a pulsed laser beam. As result of a continuous movement of the focal position through the optical waveguide 1 with a speed that could be constant or varied depending on the pattern to be obtained, micro-modifications 5 in the movement direction arise. As a result of moving the focal position through the optical waveguide 1 according to a predetermined pattern, 20 or more micromodifications 5 are generated. In a preferred exemplary example of the disclosure, more than 36 micro-modifications 5, particularly preferably more than 360 micromodifications 5 are generated by the movement of the focal position through the optical waveguide 1 according to a predetermined pattern. In a further method step, the movement of the focal position through the optical waveguide 1 is repeated according to a predetermined pattern 44.

In a further advantageous refinement, the focal position in relation to the optical waveguide 1 is modified by a translational and/or rotational movement after completion of the micro-modifications 5 introduced by the movement of the focal position through the optical waveguide 1 according to a predetermined pattern. This serves to avoid that, in the direction of the optical waveguide axis 17, the micro-modifications 5, which were introduced into the optical waveguide 1 in the repetition step by the movement of the focal position through the optical waveguide 1 according to a predetermined pattern, lie precisely behind the micro-modifications 5, which were introduced into the optical waveguide 1 in a first step by the movement of the focal position through the optical waveguide 1 according to a predetermined pattern. The movement of the focal position in the interior of the optical waveguide 1 is selected in a targeted manner dependent on a repetition rate in order to generate a predetermined arrangement of the micro-modifications 5. The method for introducing micro-modifications 5 into optical waveguides 1 may include movement of the focal position correlated to the repetition rate in such a way that an ordered uniform or systematically changing arrangement of micro-modifications 5 arises in the optical waveguide 1.

The used or achievable repetition rate of the laser system decisively determines the processing speed when introducing micro-modifications into an optical waveguide. The higher the repetition rate is, the faster the relative position between the focusing apparatus and the waveguide can be displaced in the case of an unchanging distance between the micro-modifications. Thus, a high repetition rate is preferable as a matter of principle. However, it should be noted here that the machining axes need to be displaceable in a correspondingly quick and precise manner. Furthermore, there may be heat accumulation within the optical waveguide in the case of very high repetition rates of greater than or equal to 1 MHz since the energy introduced into the irradiated volume of the optical waveguide can no longer be dissipated fast enough. This heat accumulation can lead to tension cracks and hence to mechanical instability or even to destruction of the optical waveguide. Therefore, a repetition rate is preferably selected in the range from 1 kHz to 1 MHz, particularly preferably between 1 and 100 kHz.

The use of ultrashort light pulses (pulse duration ≤10 ps) is advantageous in that the region of influence of the introduced heat energy remains very low, as a result of which the introduction of spatially restricted micro-modifications is made possible without damaging the surrounding material. If laser pulses in the nanosecond range are used, the energy from an individual pulse is transferred to the ion lattice of the irradiated material. Like in the case of a repetition rate that is too high, this also leads to heat accumulation in the irradiated material volume and to the formation of microscopic tension cracks, the extent of which by all means can lie in the millimetre range. This damage to the material of the optical waveguide can lead to restrictions in the mechanical stability, right up to a possible break of the optical waveguide under a mechanical and/or thermal load.

The use of ultrashort laser pulses allows a targeted change in the material properties only in the irradiated region of a few micrometres, without damaging regions surrounding this in an unwanted manner in the process. In this case, the pulse duration does not suffice to transfer the energy to the ion lattice of the surrounding material, and so there is no, or a strongly reduced, heat accumulation. As a result, it is possible to generate very small structures with very low tension in the surrounding material. Both the structures which are as small as possible and the tensions which are as low as possible are necessary conditions for the targeted introduction of micro-modifications into optical waveguides. Only this allows the targeted structuring of the optical waveguide material without ensuring the mechanical stability of the optical waveguide. Obtaining structures as small as possible may also be done by combining ultrashort laser pulses and controlling the focal region by obtaining a short focal region, as described previously herein. A short focal region may be obtained by controlling at least one of a short focus length or a large NA.

In a further advantageous refinement of the disclosure, the continuous movement of the focal position through the optical waveguide 1 is carried out along the optical waveguide axis and thus subsequently results in one of the described arrangements in the sectional plane. Hence, the processing procedure within a plurality of sectional planes is thus subdivided into the generation of individual points during each passage along the optical waveguide axis 17.

In a further advantageous refinement of the disclosure, the continuous movement of the focal position through the optical waveguide 1 according to a predetermined pattern is superposed with a further movement. By way of example, these movements can be vibrations which serve to establish a certain lateral offset between the micro-modifications 5, which were introduced into the optical waveguide 1 in the repetition step by the movement of the focal position through the optical waveguide 1 according to a predetermined pattern, and the micro-modifications 5, which were introduced into the optical waveguide 1 in the first step by the movement of the focal position through the optical waveguide 1 according to a predetermined pattern. Preferably, the amplitude of the vibration is at least half the distance between adjacent micro-modifications 5. Thus, an ordered arrangement of micro-modifications within the meaning of the present disclosure arises.

The micro-modifications 5 are arranged in the optical waveguide 1 in such a way that when light passes through the optical waveguide along the axis 17 of the optical waveguide, the micro-modifications are arranged in such a way that the light is deflected to the side as completely as possible by the micro-modifications.

In a further advantageous example of the disclosure, the micro-modifications 5 are introduced into the optical waveguide 1 by virtue of the optical axis 25 of the laser beam 2 being positioned off the optical waveguide axis 17 on the optical waveguide 1 when irradiating the optical waveguide 1. In the case of micro-modifications 5 whose form deviates significantly from a round form, i.e. which rather have an elongate form, this renders it possible to achieve a virtually closed surface or line of micro-modifications 5 by virtue of a rotational movement only.

In a further advantageous example of the disclosure, the micro-modifications 5 are introduced into the optical waveguide 1 by virtue of the optical axis 25 of the laser beam 2 being incident on the optical waveguide 1 at an angle (β₁, β₂, β₃) which is unequal to 90° when irradiating the optical waveguide 1. In the case of micromodifications with an elongate form, this results in an acute angle between the orientation of the micro-modification 5 and the optical waveguide axis 17. In a further refinement of the disclosure, the angle (β₁, β₂, β₃) between the orientation of the micro-modification 5 and the optical waveguide axis 17 lies in a range between 10° and 80°, in a range between 20° and 70° in a preferred refinement and between 30° and 60° in a particularly preferred refinement.

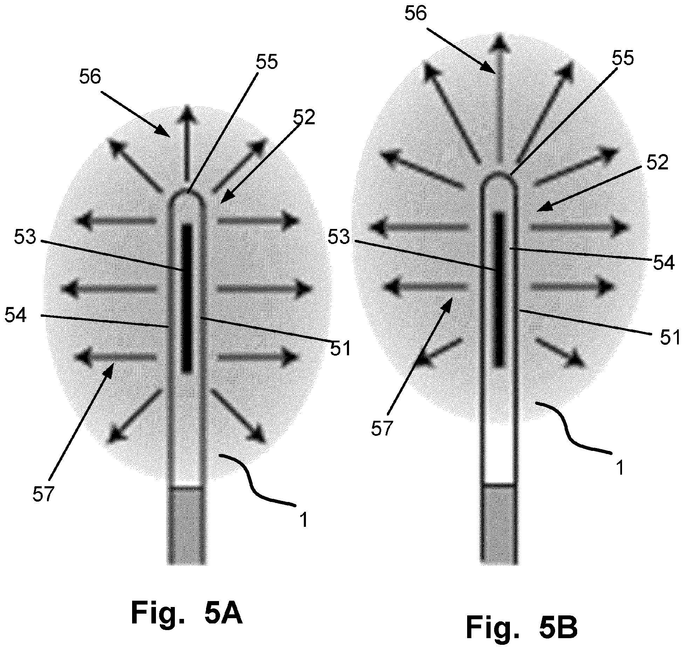

Fig. 5A and 5B are illustrating two schematic examples of optical waveguides 1, such as optical fibres. The optical waveguides 1 are adapted to be used for performing thermotherapy on at least a portion of a tissue site. Thermotherapy could here be irreversibly tissue damages for treatment purpose. The damage may be obtained by ablation through removing tissue by vaporisation, such as evaporation, or sublimation. Another example is to achieve coagulative temperatures without monitoring the progress of necrotised tissue over the time of the treatment, such as focal laser ablation (FLA), sometimes also referred to as laser-induced interstitial thermotherapy (LITT). During FLA or LITT the temperature may be monitored using MRI, ultrasound or temperature probes and temperature guards. The treatment may also be improved by obtaining an anti-tumour effect, such as an immunologic effect. The anti-tumour effect may be a local, distant or combined local/distant effect following local tumour destruction. The anti-tumour effect is triggered by antigens and may destroy any part left of a treated tumour but may also destroy other untreated tumours in the patient. Thus, the effect may be seen as a "vaccine" against a tumour (abscopal effect). The antigens are a result of a treatment causing cell death but without coagulating/denaturation of tumour antigens.

Each of the optical waveguides 1 in Fig. 5A and 5B includes a light emitting area 51 arranged at a distal portion 52. The distal portion 52 is partially a diffusor made from a structured writing. The structured writing is micro-modifications, and the micro-modifications are burnt into a core and/or cladding and/or buffer of the optical waveguide 1. The distal portion 52 of the waveguide 1 has an optical axis, and the micro-modifications are arranged along the optical axis so that light can be emitted from both a lateral surface 54 and an end surface 55 of the distal portion 52. This characteristic would make the optical waveguide 1 particularly suitable to be used in a heat probe for treatment sites where the mechanical penetration depth has to be shallower than normal.

Controlling the amount forward irradiation 56 contributes to the safety and effectiveness of the treatment especially in those cases in which the placement of the targeted area is close to anatomical structures that cannot be subject to damage otherwise causing severe adverse effects. In particular, the invention contributes to the safety and effectiveness of the treatment by enabling the treatment as close as possible to sensitive anatomical structures without the need to mechanically insert the probe as deep into tissue as would be necessary with a standard forward irradiation profile. The risk of mechanical damage to these structures can thereby be minimised without compromising the size of treated tissue close to these sensitive structures. This result is achieved because the design allows a controlled deposit of energy beyond the tip of the heating probe. The named deposit of energy can be tailored to a given application by varying the density and distribution of the micro modifications within the core.

Examples of applications in which the feature is important can be found in neurosurgical applications such treatment of tissue where the tissue is brain tissue, for treatment of a neurological disease, such as treatment of tumors, brain, radiation necrosis, neurological functional disorders, Parkinson's disease or epilepsy centres in close proximity of eloquent structures, where a mechanical damage or an uncontrolled thermal spread may lead to functional or sensory impairment or even death, or treatment of suicidal tendencies, or other treatment sites such as urology applications such as treatment of tumorous tissue adjacent to the prostate capsule, where a perforation of the capsule may lead to fistula formation and potentially sepsis or nerve damage. The invention is particularly effective if combined with non-invasive real time temperature monitoring using for example MR thermometry.

The arrangement of the micro-modifications to allow light not only to be emitted from a lateral surface 54 of the optical waveguide 1, but also from an end surface 55, makes it possible to treat patients and tissue locations not normally available for this type of treatment. In particular, the herein described arrangement of the micro-modifications which allows light to be emitted from an end surface 55 provides the advantage that the heat probe can be position shallower in the tissue than a traditional heat probe using light. For treatment of brain tissue, such as neurological disease, a suitable emittance of diffused light through the end surface 55 the diffusing optical waveguide end is about 5% to 25%, such as 5% to 15%, such as 8% to 12%, such as 10% to 25%, such as 15% to 25%, such as 20% to 25% of the total light being emitted from the diffusing optical waveguide tip or light emitting area 51. In other applications could a suitable emittance of diffused light through the end surface 55 of the diffusing optical waveguide be about 40% to 50%.

To control the light profile emitted for the optical waveguide tip or light emitting area 51, both from the lateral surface 54 and the end surface 55, the micro-modifications are varied along the optical axis. For example, the density of the micro-modifications may increase towards the light emitting end surface 55 to compensate for power losses in the light when propagated along the optical axis. This may achieve a flat top profile along the radial emission 57. The density of the micro-structures may vary by changing the distance between the micro-modifications along the optical axis. The density may increase by shortening the distance between the micro-modifications in the direction of the optical axis. The difference in density over the length of the diffuser 53 may be fairly large and go from 5 to 60 U/mm or from 5 to 40U/mm, where U is the number of micro-modifications. Other ranges of density may be required since the range may depend on the length of the diffuser. A shorter diffuser 53 may require a smaller range than a longer diffuser 53 to compensate for the loss in light power closer to the light emitting end surface 55 of the optical waveguide 1 due to the light propagating through the diffusor 53. Depending on the wanted light profile of the diffuser 53, the variation of the micro-modifications may not only involve an increase of the density towards the light emitting end surface 55 but could also be that there are sections of increased and decreased density spread out along the optical axis. The diffusor 53 may have different lengths depending on the application, but common lengths are between 5 to 30mm, such as 10 to 30mm, such as 15mm, such as 20mm such as 25mm. Additionally or alternatively to change the density of the micro-modifications may be to change the scattering strength by changing the scattering properties of the micro-modifications, such as the size or shape of the micro-modifications.

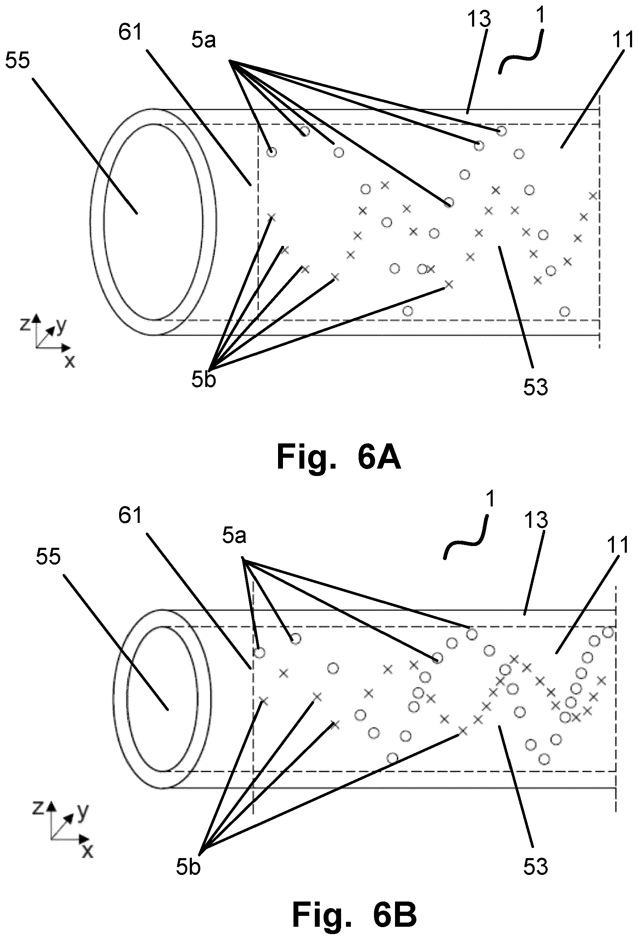

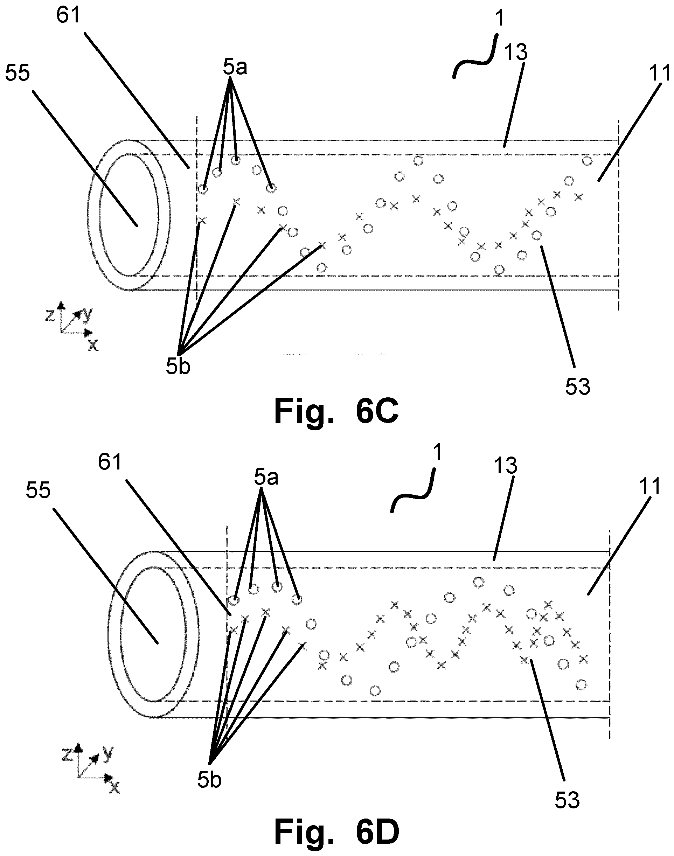

Figs. 6A to 6H illustrate schematic examples of micro-modifications 5a, 5b arrangements in an optical waveguide 1. The optical axis is along the X-axis. The micro-modifications 5a, 5b are arranged as a helix or spiral along the optical axis starting at position 61. This position is usually close to the light emitting end surface 55 of the waveguide 1. The starting position 61 of the structures made from micro-modifications 5a, 5b may be arranged at a distance from the light emitting end surface 55 of the waveguide, such as 1 to 5mm, such as 2mm, such as 1 mm. When manufacturing the diffusing region 55 of the waveguide 1, a certain position is selected as the starting position 61 of the structures off micro-modifications 5a, 5b. When the structures have been created, the distance between the light emitting end surface 55 of the waveguide 1 and the starting position 61 of the structures may be obtained by cutting the waveguide 1 at a selected distance from the beginning of the diffuser 53. In some examples may the laser source used for introducing the micro-modifications 5a, 5b be used to cut the waveguide 1, such as automatically cut the waveguide 1 after the structures have been introduced into the core 11 or cladding 13 and/or a coating/buffer of the waveguide 1. The laser source may also be used to cut the waveguide 1 an end opposite the light emitting end 55. The micro-modifications 5a, 5b may be arranged as at least two helices or spirals, as illustrated in figure 6A, wherein each spiral or helix has a different radius. Each spiral or helix may have a constant pitch and the micro-modifications are positioned on the spiral or helix with a constant distance separating the micro-modifications. The at least two spirals or helices may have different pitches or the same pitch. The distance between the micro-modification 5a, 5b on each spiral or helix may in some examples be the same or in some examples may the distance between the micro-modifications 5a, 5b differ between the spirals or helices. The spirals or helices may have different translation (phases) as illustrated in Fig. 6A or have substantially the same translation (phase) as illustrated in Fig. 6C. Alternatively, the at least two spirals or helices may have the same radius and pitches but differing by a translation (phase) along the optical axis.

Similar to Fig. 6A, Figs. 6B to 6D are illustrating diffusers 53 having at least two spirals or helices. In Fig. 6B, the density of the micro-modifications 5a, 5b is varied along the optical axis by changing the distance between succeeding micro-modifications 5a, 5b arranged on each spiral or helix, i.e the distances between succeeding micro-modifications 5a, 5b arranged on at least one of the spirals or helices may increase or decrease along the optical axis to obtain a light emitting profile with a required characteristic, including a controlled amount of light being emitted from the distal end surface 55. The distances between succeeding micro-modifications 5a, 5b, may be varied on each spiral or helix individually such that the distances between succeeding micro-modifications 5a, 5b, are only increasing or decreasing on one of the at least two spirals or helices, the distances may increase on one spiral or helix and decrease on another spiral or helix, the distance may increase or decrease on more than one spiral or helix but with different amount.

In Fig. 6B both spirals or helices have decreasing density of succeeding micro-modifications 5a, 5b along the optical axis in a directed towards the emitting end surface 55. The density is decreased by increasing the distances between succeeding micro-modifications 5a, 5b on both spirals or helices. In other examples may both spirals or helices have increasing density of succeeding micro-modifications 5a, 5b along the optical axis in a directed towards the emitting end surface 55. The density is increased by decreasing the distances between succeeding micro-modifications 5a, 5b on both spirals or helices. In some further examples may one of the spirals or helices have increasing density of succeeding micro-modifications 5a, 5b along the optical axis in a directed towards the emitting end surface 55 while the other spirals or helices have decreasing density of succeeding micro-modifications 5a, 5b along the optical axis in a directed towards the emitting end surface 55.

Fig. 6D is illustrating an addition and/or alternative to vary the density of the micro-modifications 5a, 5b along the optical axis. In 6D, the pitch may change so that the distance covered by one complete rotation of a spiral or helix is either increasing or decreasing, such that the distance for a complete rotation is either longer or shorter. By changing the pitch on one or more of the spirals or helices, the density of the micro-modifications 5a, 5b increases and decreases which may be used to control the profile of the emitted light.

To control the density of the micro-modifications5a, 5b, a combination of what has been described herein with respect to figures 6B and 6D may be used. Additionally, and/or alternatively, the at least two spirals or helices may not be continuous along the whole diffuser 53 section of the optical waveguide. There may be sections of the diffuser 53 with only a single spiral or helix combined with sections with at least two spirals or helices, which may also affect the density of the micro-modifications 5a, 5b along the optical axis of the waveguide 1.

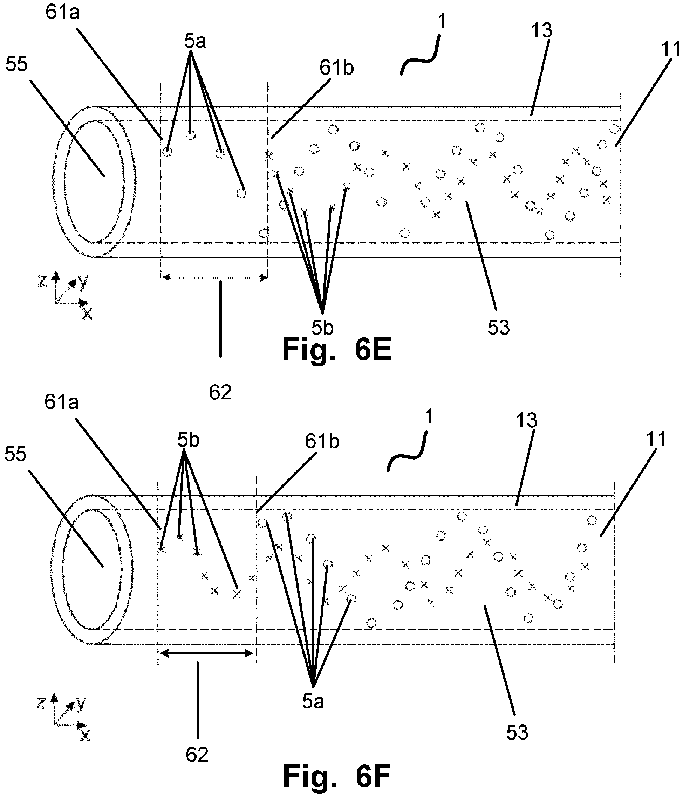

Fig. 6E to 6H are illustrating further arrangements of the micro-modifications 5a, 5b. The examples illustrated may be combined with the arrangement of micro-modifications 5a, 5b described in relation to Figs. 6A to 6D. In Figs. 6E and 6F, the at least two helices and spirals are arranged with an offset 62 in relation to the starting point 61a, 61b, of each spiral or helix in a direction of the optical axis. In Fig. 6E, the outer spiral or helix comprising micro-modifications 5a has an earlier start point 61a then the inner spiral or helix comprising micro-modifications 5b. In Fig. 6F the opposite is illustrated, the inner spiral or helix, comprising micro-modifications 5b, has an earlier staring point 61a then the outer spiral or helix comprising micro-modifications 5a with a starting point 61b. between the two starting points 61a, 61b there is an offset 62 in the direction of the optical axis.

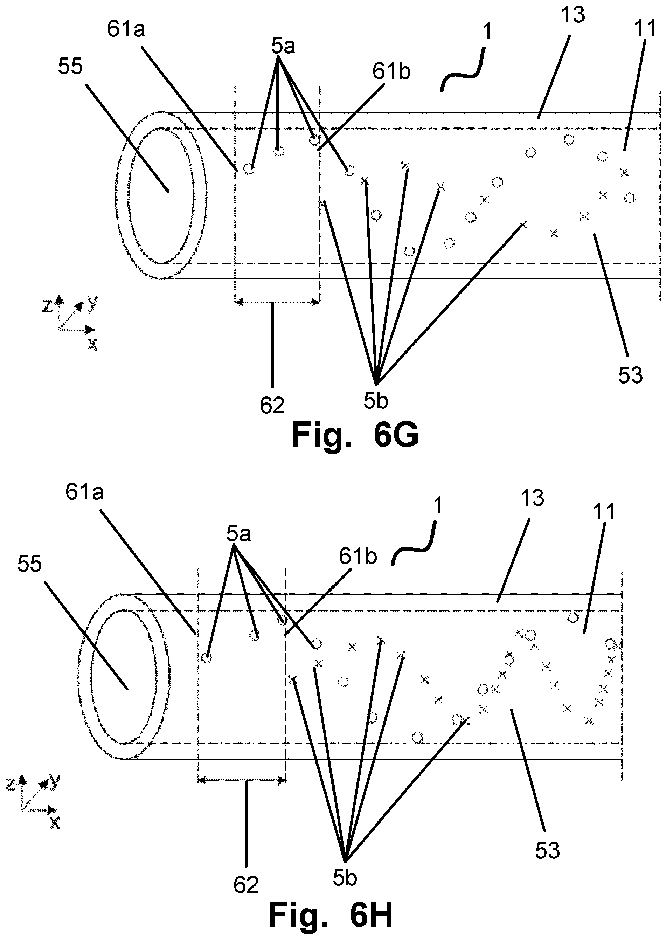

Fig. 6G is illustrating an offset 62 between starting points 61a, 61b of the outer and the inner spirals or helices similar as in Fig. 6E, with a translation (shift in phase) along the optical axis. Fig. 6H is similar to the arrangement in Fig. 6G combined with a change in the pitch, wherein the inner spiral or helix has a decrease in the pitch moving along the optical axis away from the light emitting light surface 55, and a decrease in the distance between succeeding micro-modifications which means that the density of the micro-modification will vary along the optical axis, in this particular example, the density is increasing in a direction away from the light emitting surface 55. The illustrated arrangement of the micro-modifications 5a, 5b may be arranged in an opposite fashion so that the increased density is in a direction towards the light emitting end surface 55. However, other arrangements of the micro-modifications 5a, 5b are possible as described in relation to Figs. 6A to 6F.

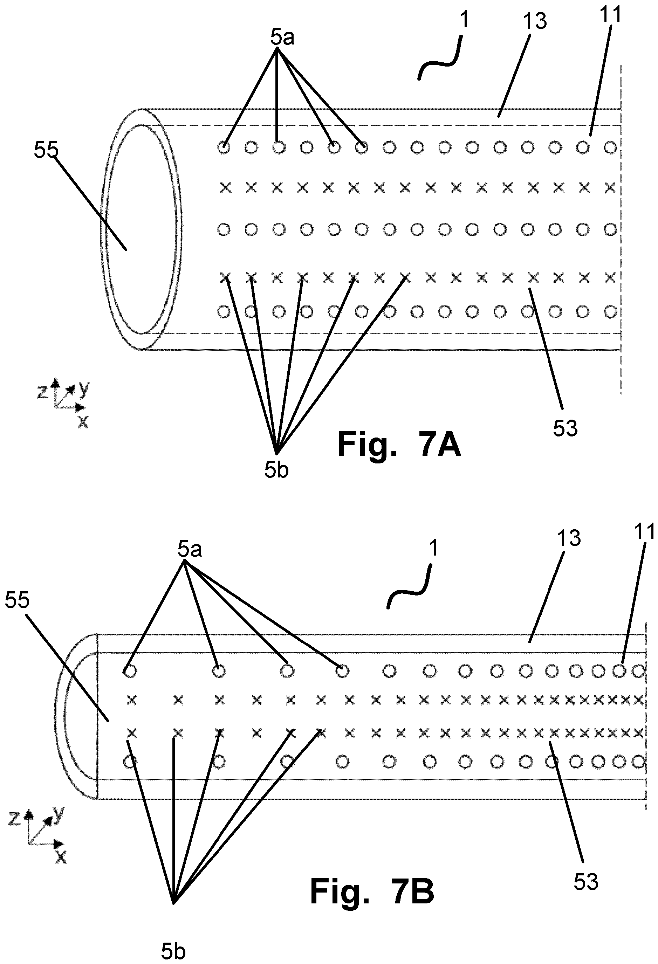

Figs. 7A to 7D are illustrating an alternative to using spirals or helices. In these examples lines of micro-modifications 5a, 5b are introduced. The lines are introduced with at least two different distances to the optical axis. Each line is parallel to the optical axis and for each distance to the optical axis a plurality of lines are arranged forming a structure having a tube like resemblance. For each distance to the optical axis, the lines of micro-modification 5a, 5b may have the same distance between succeeding micro-modifications 5a, 5b, as illustrated in Fig. 7A. Further, the number of distances for which lines are arranged may vary along the optical axis of the light diffuser, such as for certain sections of the light diffuser 53 lines are only arranged at one distance from the optical axis and for other sections of the light diffuser 53, lines are arranged on at least two distances form the optical axis. In some examples may the number of lines of micro-modification 5a, 5b vary for each distance from the optical axis along the optical axis of the light diffuser 53.

Fig. 7B is illustrating an arrangement of micro-modifications 5a, 5b arrange in lines, similar as to Fig. 7A. In Fig. 7B, the density of the micro-modifications 5a, 5b is varied along the optical axis of the light diffuser 53. In the illustrated example, the distance between succeeding micro-modifications 5a, 5b is decreasing towards the light emitting end surface 55, i.e. the density is increasing. The increase in the density is illustrated for lines of micro-modifications 5a, 5b arranged at both distances to the optical axis. Alternatively, only micro-modifications 5a, 5b arranged along lines at one of the distances from the optical axis may have an increase or decrease of the distances between succeeding micro-modifications 5a, 5b.

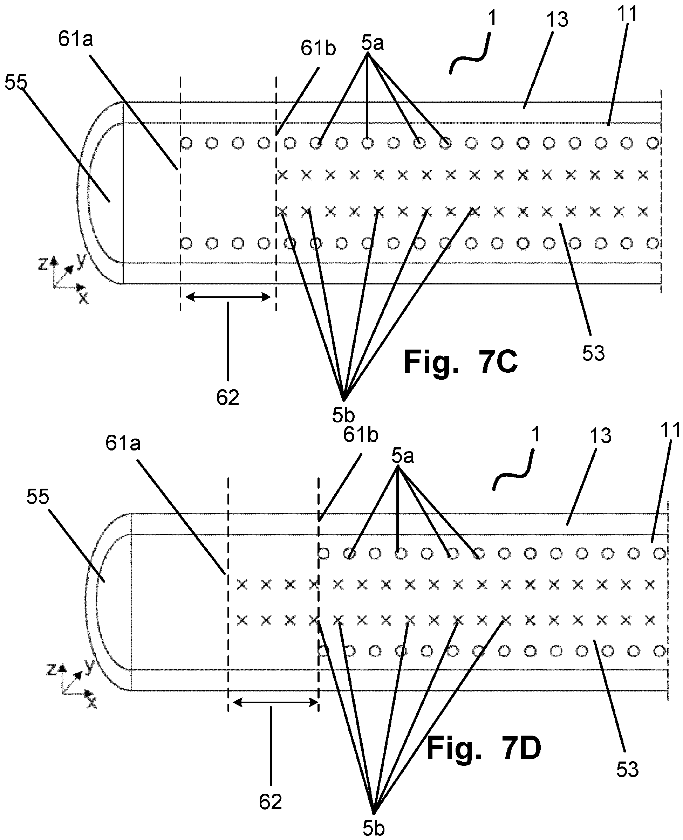

Figs. 7C and 7D are illustrating examples where there is an offset 62 between starting points 61a, 61b of the lines of micro-modification 5a, 5b arranged at the two different distances from the optical axis. In Fig. 7C the starting point 61a for the lines of micro-modifications 5a with the longest distance to the optical axis is earlier than the starting point 61b for the lines of micro-modification 5b closer to the optical axis. Fig. 7D is illustrating the reverse arrangement where the starting point 61b for the lines of micro-modifications 5b with the closest distance to the optical axis is earlier than the starting point 61a for the lines of micro-modification 5a farther away from the optical axis.

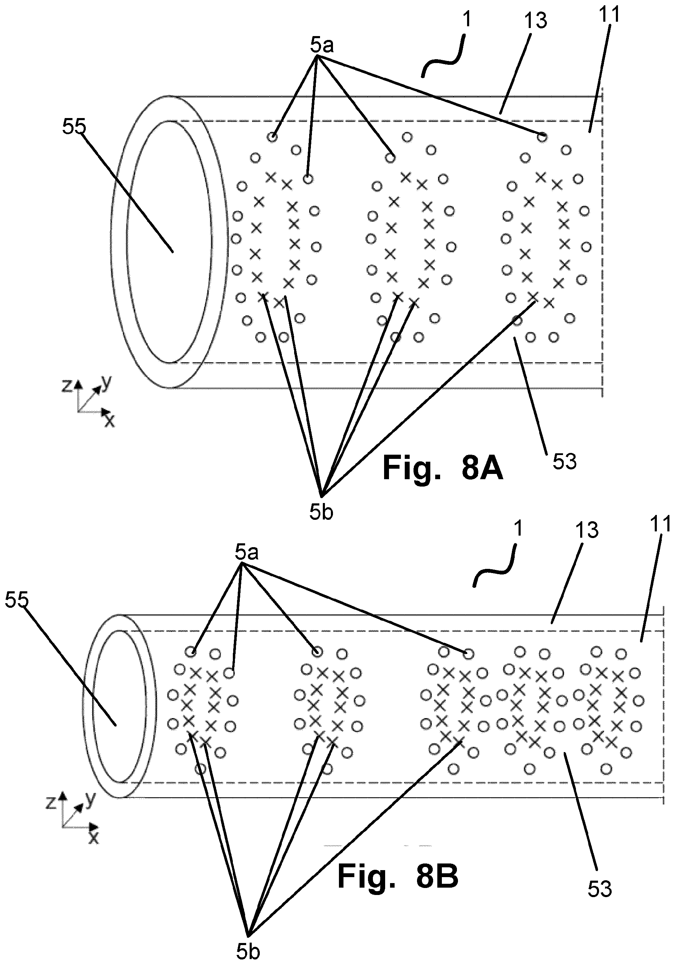

Figs. 8A to 8G are illustrating micro-modifications 5a, 5b arranged in circles along the optical axis of the optical waveguide. In Fig. 8A, at each position there are two circles of micro-modifications 5a, 5b arranged, one with a larger radius and one with a smaller radius. The circles are concentric with the optical axis of the waveguide. Each circle formed for each radius is illustrated as having the same number of micro-modifications 5a, 5b, such as each circle with the smaller radius has the same number of micro-modifications 5b and each circle with the larger radius has the same number of micro-modifications 5a. The inner and outer circle may have the same number of micro-modifications 5a, 5b or the number of micro-modifications 5b in the inner circles may differ from the number of micro-modifications 5a in the outer circles. The distances between each pair of circles are constant so that the circles are evenly arranged over the length of the light diffuser 53.

Fig. 8B illustrates a similar arrangement as Fig. 8A with the difference that the distance between each paid of circles of micro-modifications 5a, 5b are varied. In the example illustrated in Fig. 8B, the distance between each pair of circles is increasing towards the light emitting end surface 55 which leads to a decrease in the density of micro-modifications 5a, 5b towards the light emitting end surface 55. The arrangement of circles can also be made so that the distance between each pair of circles is decreasing towards the light emitting end surface 55 which leads to an increase in the density of micro-modifications 5a, 5b towards the light emitting end surface 55.

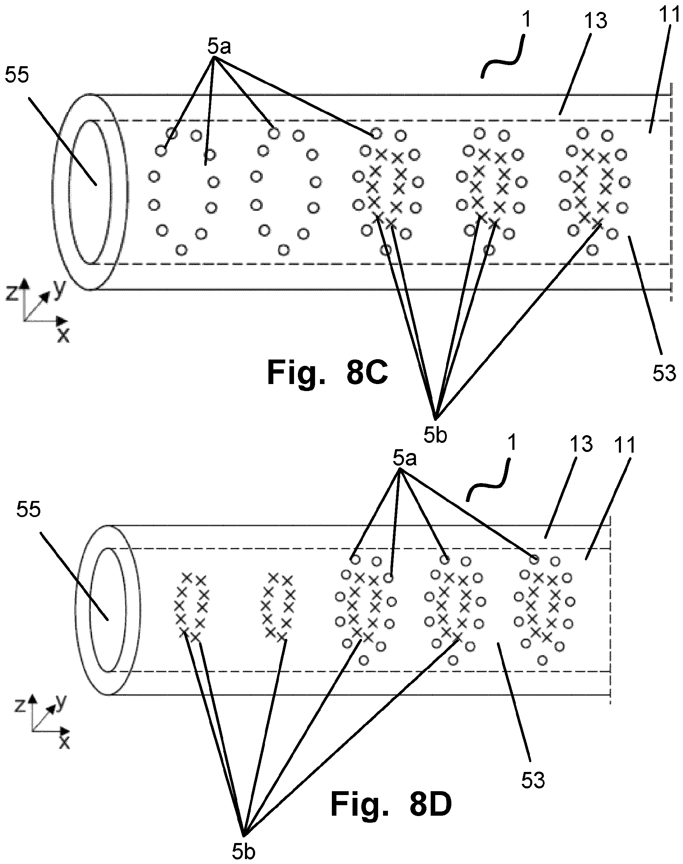

Figs. 8C and 8D are illustrating a similar arrangement as in Fig. 8A but with an offset between the inner circles of micro-modifications 5b and the outer circles of micro-modifications 5a. In Fig. 8C, the starting point for the outer circles of micro-modifications 5a is earlier than the starting point for the inner circles of micro-modifications 5b. The distance between each pair of circles may be constant or vary, such that the distances between two pairs of circles of micro-modifications 5a, 5b are increasing or decreasing. In Fig. 8D the starting point for the outer circles of micro-modifications 5a is after than the starting point for the inner circles of micro-modifications 5b. The distance between each pair of circles may be constant or vary, such that the distances between two pairs of circles of micro-modifications 5a, 5b are increasing or decreasing.

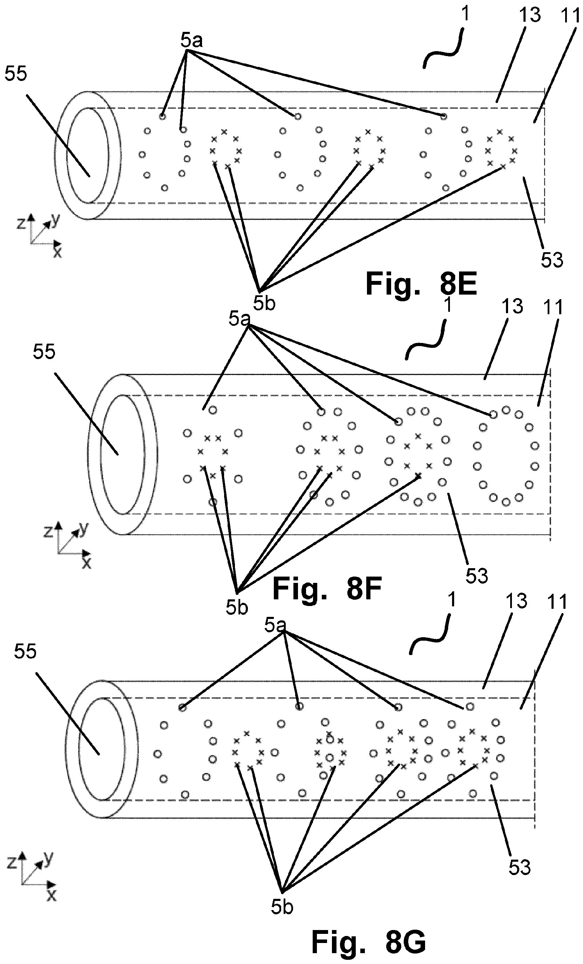

In Fig. 8E, the circles of micro-modifications 5a, 5b are not arranged as pairs as in the previous Figs. 8A to 8D, instead the circles are offset so that every other circle is a large circle of micro-modifications 5a and between two larger circles of micro-modifications 5a is a smaller circle of micro-modification 5b.

Fig. 8F is illustrating an example of an arrangement where the density of micro-modifications 5a, 5b are varied by increasing number of micro-modifications 5a in the outer circle of each pair of circles with micro-modifications 5a, 5b away from the light emitting end surface 55 of the optical waveguide 1 and the distance between each pair of circles are decreasing. In some arrangement, the density of micro-modifications 5a, 5b are varied by increasing number of micro-modifications 5a in the outer circle of each pair of circles with micro-modifications 5a, 5b towards the light emitting end surface 55 of the optical waveguide 1 and the distance between each pair of circles are decreasing.

Fig. 8G is illustrating an offset similar of Fig. 8E with the addition of a variation of the number of micro-modifications 5a, 5b in each circle at each distance from the optical axis. The distance between the inner circles of micro-modification 5b is decreasing away from the light emitting end surface 55 leading to an decrease in density of the micro-modifications 5a, 5b closer to the light emitting end surface 55. In some examples is the distance between the inner circles of micro-modification 5b increasing away from the light emitting end surface 55 leading to an increase in density of the micro-modifications 5a, 5b closer to the light emitting end surface 55.

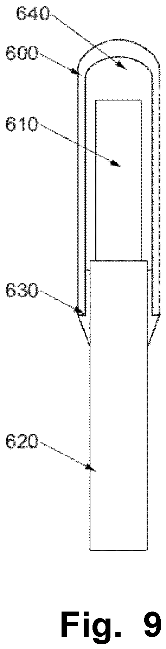

Fig. 9 is illustrating a capillary to be used to protect the emitting area of an optical waveguide, in this example, an optical fibre. The emitting area of the optical waveguide is a diffuser. The diffuser may be any of the examples provided herein and schematically illustrated in Figs. 5A to 5B, 6A to 6H, 7A to 7D and 8A to 8G.

The optical waveguide, which may be an optical fibre, may be, for example, made of silica or a plastic, or may be any other suitable type of optical waveguide. The optical waveguide may also be a polymer coated waveguide.

Fig 9 illustrates a capillary 600 covering an end of a waveguide 610 which may include a diffuser section. The waveguide 610 and the diffuser section may be any type of waveguide and diffuser section described herein. The diffuser section of the waveguide 610 comprises of a waveguide core and may also in some examples include at least one cladding. The diffuser section may also include at least one jacket, coating and/or buffer. The tip of the distal end of the waveguide 610 may be in some examples flat as illustrated in Fig. 9 or conical. The capillary 600 may be made from glass or plastic. The capillary may be bonded 630 to the jacket 620 and/or buffer of the waveguide either by fusing or by using an adhesive, and/or by shrink tubing. The space 640 around the bare end fibre 610 may be filled with air. This design of the capillary increases the waveguide's mechanical stability and its resistance to high temperatures. The heating probe itself may comprise the jacket of which the optical fibre is made from, for example acrylate, then a layer of, for example, resin and an outer layer made of, for example, PBT or PTFE.

When using laser light to heat and ablate tissue most diffuser fibres use a cooling system with the dual purpose of i) protecting the integrity of the probe from high temperatures in the internal structures and ii) avoid carbonisation in tissue due to high temperatures in the interface between the probe and treated tissue. If a cooling system is not used, the low temperature resistance in most diffuser designs, will restrict the maximum laser power and thereby only small ablation sizes can be achieved. When controlling the laser light power there are some clear disadvantages using cooling, e.g. increasing the treatment time and creating a risk of leaving viable tissue close to the probe. It is also more difficult to control a treatment when energy is both deposited, laser light, and removed, cooling media. Using a cooling media also introduce a patient risk if supply of cooling media is interrupted which will destroy the probe's internal structure leading to uncontrolled emission of laser power in tissue as well as leakage of cooling media into tissue. In a neuro application both these events may be catastrophic for the patient and in other applications this may lead to unintentional tissue destruction.

To achieve a safe treatment, using a non-cooled probe, without the risk of structural damage to the probe, the materials in the probe must withstand high temperatures well over 100 ºC. Further to avoid tissue carbonisation at high laser power levels the energy density must be restricted. The current invention overcomes these limitations by using only high temperature resistant materials (protection against structural damage) and by using a capillary that covers the internal structure of the diffuser element. The capillary protects the internal structure from mechanical damage as well as lowers the energy density in the interface between the probe and tissue. For the same laser power, the energy density decreases as the square of the capillary radius. The combination of micro modification in a silica fiber and using a silica capillary, as in Fig. 9, makes it possible to design an un-cooled probe that can deliver high laser power into tissue.

Providing an un-cooled probe removes the need of a cooling system, reduces the complexity of the treatment procedure and of the devices involved in the treatment. When using a cooled solution, especially when the treatment is performed with MR guidance and/or MR thermometry, special installations may be required to be able to handle the cooling fluid. The non-cooled applicator does not require the use of fluid circulation devices and requires only a simplified setup if compared to a cooled solution, no tubing needs to be routed into the sterile field or in the MR room and no circulation system is required.

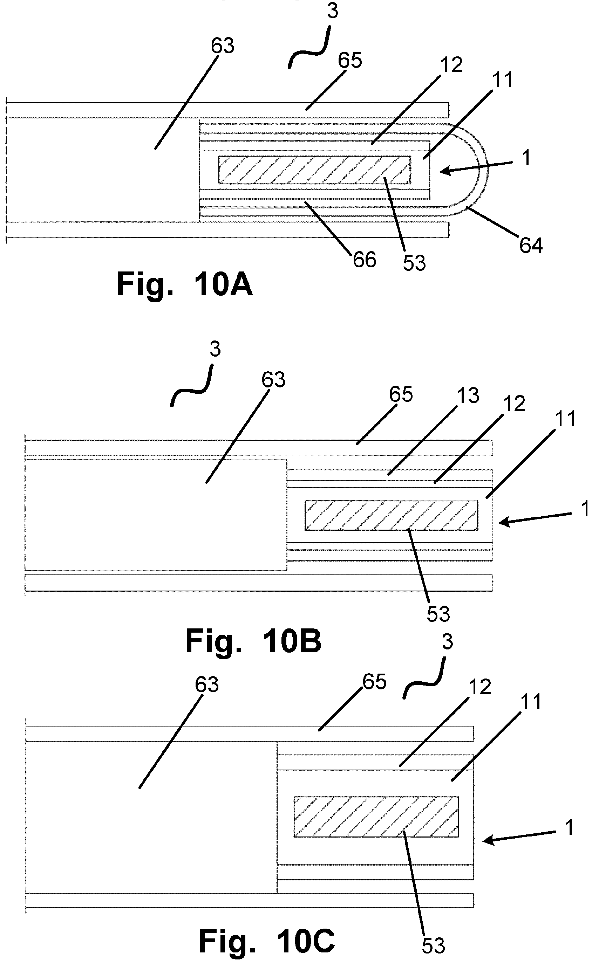

Further schematic examples of probes 3 are illustrated in Figs. 10A to 10G. Fig. 10A is illustrating a capillary 64 covering an end of a waveguide 1, which may be an optical fibre. A distal end of the waveguide 1 may include a diffuser 53. The diffuser section 53 of the waveguide 1 comprises of a waveguide core 11 and may also in some examples include a cladding 12. The capillary 64 may be made from glass or plastic. A shrink tubing 65 is arranged outside a jacket 63 of the optical waveguide 1 and along an external surface of the capillary 64. The shrink tubing 65 may extend partially along the outer surface of the capillary 64 or extend along the whole outer surface of the capillary 64 only leaving the distal end uncovered. Alternatively, the shrink tubing 65 may be configured like a sock with a closed end. The shrink tubing 65 may hold the capillary 64 which means that no additional adhesive, glue or fusing is required for attaching the capillary 64 to the heating probe 3. The shrink tubing 65 is made from a thin material. The shrink tubing 65 may be transparent to the wavelength emitted by the diffuser 53. At the distal end of the end of the waveguide, where the emitting area is, a space 66 between the waveguide 1 and the capillary 64 may be filled with air.

Fig. 10B is illustrating probe 3 which includes a distal end of the waveguide 1 which may include a diffuser 53. The diffuser section 53 of the waveguide 1 comprises of a waveguide core 11 and may also in some examples include a cladding 12. A shrink tubing 65 is arranged outside a jacket 63 of the optical waveguide 1 and along an external surface of the diffuser section 53 of the waveguide 1. The shrink tubing 65 may extend partially along the outer surface of the diffuser section 53 or extend along the whole outer surface of the diffuser section 53 only leaving the distal end uncovered. The shrink tubing 65 may protect the diffuser section 53 of the waveguide 1 which has been stripped from other layers, such as the outer jacket 63. In the illustrated example, the buffer coating 13 is left, at least partially, for extra protection. The shrink tubing 65 is made from a thin material which may be transparent to a wavelength 1 emitted by the diffuser 53.

Fig. 10C is illustrating an example of a probe 3 similar to Fig. 10B with the difference that there is no buffer coating, thus the diffuser section 53 is only the core 11 and the cladding 12 of the waveguide 1.

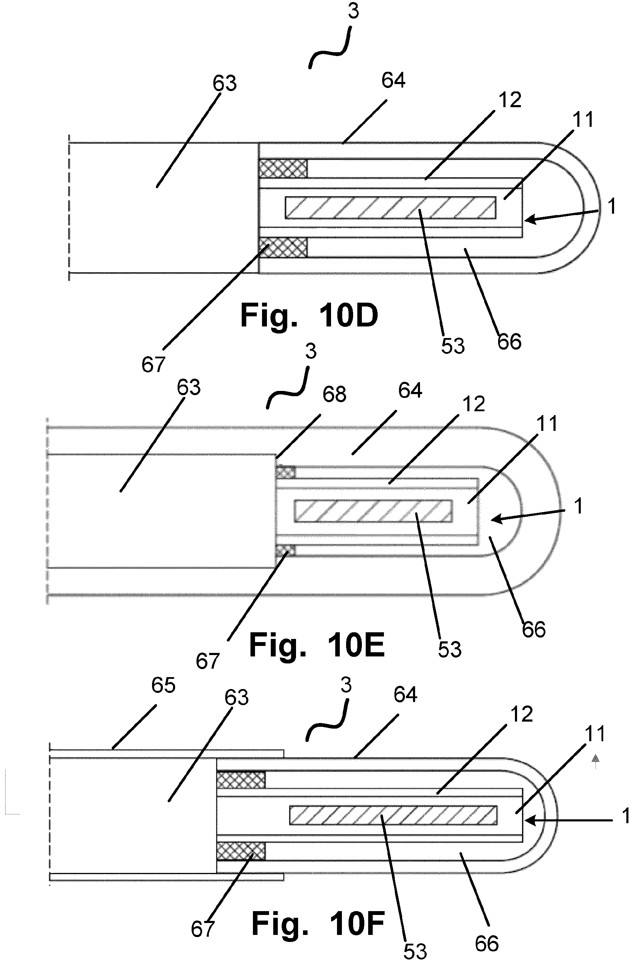

Fig. 10D is illustrating a probe 3 which includes a distal end of the waveguide 1 which may include a diffuser 53. The diffuser section 53 of the waveguide 1 comprises of a waveguide core 11 and may also in some examples include a cladding 12. In some further examples, the diffuser section 53 may also include the buffer coating. The capillary 64 may be made from glass or plastic. In the illustrated example, the capillary 64 is substantially aligned with the jacket 63 of the optical waveguide 1 and attached by an adhesive 67, such as a glue, applied between the diffuser section 53 and the capillary 64. The adhesive 67 is arranged at a distal and of the jacket member 63 which will be at a proximal end of the capillary 64. At the distal end of the end of the waveguide 1, where the emitting area is, a space 66 between the waveguide 1 and the capillary 64 may be filled with air.

Fig. 10E is illustrating probe 3 which includes a distal end of the waveguide 1 which may include a diffuser 53. The diffuser section 53 of the waveguide 1 comprises of a waveguide core 11 and may also in some examples include a cladding 12. In some further examples the diffuser section may also include the buffer coating. The capillary 64 may be made from glass or plastic. In the illustrated example, the capillary 64 is arranged to extend over the jacket member 63 of the optical waveguide 1. The capillary 64 may extend over the jacket member 63 from a few mm up to a couple of cm, such as 50mm. The capillary 64 may be thicker in the section covering the diffuser section 53. This is made for minimising the space 66 between the capillary 64 and the diffuser section 53. In these examples, there may be a step 68 in the capillary 64 which is aligned with the distal end of the jacket member 64. The capillary is attached to the optical waveguide 1 by an adhesive 67, such as a glue, applied between the diffuser section 53 and the capillary 64. The adhesive 67 is arranged at a distal and of the jacket member 63. At the distal end of the end of the waveguide 1, where the emitting area is, a space 66 between the waveguide 1 and the capillary 64 may be filled with air.

Fig. 10F is illustrating a probe 3 which has a capillary 64 covering an end of a waveguide 1, which may be an optical fibre. A distal end of the waveguide 1 may include a diffuser section 53. The diffuser section 53 of the waveguide 1 comprises of at least a waveguide core 11 and may also in some examples include a cladding 12. In some further examples, the diffuser section 53 may also include a buffer coating. The capillary 64 may be made from glass or plastic. A shrink tubing 65 is arranged outside a jacket member 63 of the optical waveguide 1 and partially along an external surface of the capillary 64 The shrink tubing 65 may extend along the outer surface of the capillary 64 with a fem mm, such as 5 to 15mm, such as 5 to 10mm. The shrink tubing 65 may extend up to 40 cm over the jacket member 63 to keep a straight section of the waveguide 1. The shrink tubing 65 may assist in holding the capillary 64. In the illustrated example, the capillary 64 is substantially aligned with the jacket 63 of the optical waveguide 1 and attached by an adhesive 67, such as a glue, applied between the diffuser section 53 and the capillary 65. The adhesive 67 is arranged at a distal and of the jacket member 63 which will be at a proximal end of the capillary 64. At the distal end of the end of the waveguide 1, where the emitting area is, a space 66 between the waveguide 1 and the capillary 64 may be filled with air.

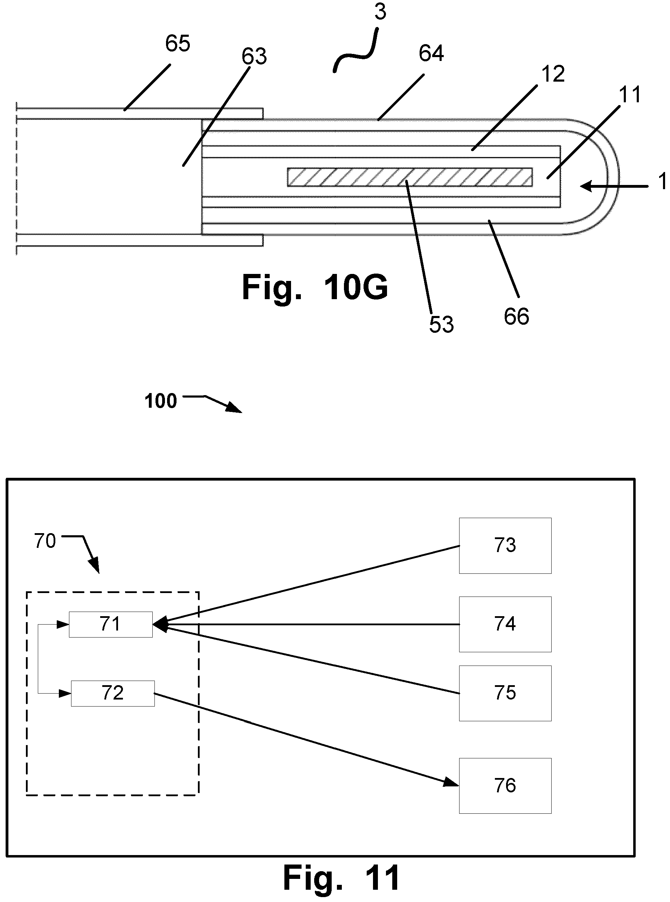

Fig. 10G is illustrating an example of a probe 3 similar to Fig. 10F with the difference that there is no additional adhesive used for attaching the capillary 64. Thus, the capillary 64 is attached to the probe 3 only using the shrink tubing 65 as in Fig. 10A.

Fig. 11 illustrating schematic treatment systems. The systems are configured for controlling a thermotherapy of tissue. The thermotherapy is controlled by controlling a size of a treatment lesion covering at least a portion of tissue to be treated. Preferably the treatment lesion is sized to the whole tissue area to be treated, by positioning the temperature measuring element used for controlling the treatment outside the tissue area. Preferably the temperature measuring element used for controlling the treatment is positioned about 0 to 5 mm, such as 2 to 5mm, outside the boundary of the tissue to be treated, i.e. 0 to 5 mm, such as 2 to 5mm, outside the treatment lesion. The size of the treatment lesion may thereby be determined by the distance between the energy emitting part of the probe and the point in the tissue where the temperature is selected to reach a target temperature, such as a target temperature between 40 to 60°C. In particular, the disclosure relates to a device, system, and method for obtaining coagulative temperatures and accurately monitoring the progression of necrotised tissue over time or obtaining an anti-tumour immunologic response by thermotherapy of at least a portion of a tumour. As an alternative to temperature measuring element may other modalities be used for monitoring the temperature, such as MRI, ultrasound etc. This may be the same modality used for investigating the treatment site and the surrounding tissue for establishing a size and shape of the treatment site. Thus, may be done using ultrasound, MRI or other suitable imaging modalities.

In an example according to Fig. 11, a schematic illustration over an exemplary system 100 for thermotherapy of tissue, such as a tumour, is illustrated. The system 100 is especially developed for controlling the thermotherapy by controlling the size of a lesion covering at least a portion of a treatment site. The system 100 may in some examples be used for controlling immunostimulating laser thermotherapy.

The system 100 comprises a control unit 70 which includes a temperature reading unit 71 configures to receive temperature data from a temperature measuring element 73. The temperature measuring elements 73 may be a temperature sensor, such as a thermistor/thermocouple that are used as temperature probes and/or temperature guards. The control unit 70 may be a computer, a microprocessor or an electronic circuit for converting an input signal to an output signal. The control unit 70 may for example be performed using a feed-back loop. In some examples of the system 100, at least one of the temperature measuring element 73 may be arranged outside the treatment site for measuring a temperature which can be used to control the treatment.

In some examples of the system 100, at least one of the temperature measuring element 730 may be arranged in a sleeve of the treatment probe. The temperature measuring element 73, such as thermistor/thermocouples, may be arranged in a channel of the sleeve, such as a multi-lumen sleeve. The sleeve may be heat shrunk after the temperature measuring elements have been arranged in the channels of the sleeve. Alternatively, in some examples, the sleeve may comprise two shrink tubing concentrically arranged. The temperature measuring elements 73 may be arranged between the two tubes. The tubes may thereafter be heat shrunk. Another alternatively, in some example, the temperature measuring element 73 may be braided or woven into the sleeve.

Alternatively and/or additionally to have the temperature sensors arranged in the sleeve or having separate thermo-probes and/or guards, Magnetic Resonance Imaging may be used to obtain a 2D or 3D temperature maps of the treatment area. A region of interest (ROI) may be defined by determining a point at a distance, such as at the tumour boarder, from the emitting area. This region will define a treatment lesion and temperature in this ROI may be used to control the heat source in order to maintain the temperature at a predetermined value. Other regions in the 2D temperature map of the MR Image may also be defined to serve as an automatic safety function shutting down the heat source if a threshold temperature is reached.

Close to the tip of the heating probe 76 is a light emitting area for emitting light from the optical fiber for heating a treatment lesion. Energy, in the example light of a certain power, is emitted from the emitting area of the heating probe 76, in operation thereof, for the heating of the lesion. The probe is preferably in apposition with the treatment lesion, or at least positioned at the treatment lesion. The treatment lesion may cover at least a portion of a site to be treated. The covered portion of a treatment site has a boundary adjacent healthy tissue surrounding the site. Additionally, the heating probe may emit diffused light from the emitting area. The diffused light may be provided by fitting a distal end of the optical fiber with a light diffusor.

In the illustrated example only one heating probe 76 is shown, but depending on the size and shape of the treatment site to be treated more than one heating probe may be used.

Additionally to a heating probe 76, the apparatus 100 may include a further thermal sensor member 74, such as a high temperature probe, for measuring the temperature in the proximity to an emitting area of the heating probe 74. The further thermal sensor member 74 is connected to the control unit 71.

Additionally and/or alternatively, under some conditions, the temperature measured by the first thermal sensor member 73 may be used to estimate the temperature of the tissue a defined distance from the treatment lesion or a temperature at a boundary between the at least a portion of the treatment site and surrounding healthy tissue. This could be done by obtaining a temperature gradient, e.g. how much the temperature changes per millimetre, by running computer simulations (i.e Monte Carlo, finite elements method or ray tracing based on optical properties). Alternatively and/or additionally, in some examples, a first thermal sensor member 73 having multiple measuring points spaced with a known distance may be used to estimate the thermal gradient.

Additionally to the first thermal sensor member 73 and/or a further thermal sensor member 74, in some examples, a further thermal sensor member, or a plurality of such thermal sensor members, may be used as a guard thermal sensor member 75. A guard thermal sensor member 75 may be positioned, as a precaution, in close proximity to a sensitive area of an organ to avoid damages due to heat. A sensitive area could be anywhere either inside the treatment lesion or outside of the lesion.

The sensors for measuring the temperature in the first thermal sensor member 73, a further thermal sensor member 74 and guard thermal sensor member 75 may, for example, be thermistors, thermocouples or a fiber bragg gratings (FBG).

Additionally and/or alternatively, in some examples any of the thermal sensor members 73, 74, 75 may have a single measuring point or multiple sensors for measuring at multiple points

Alternatively, instead of using punctuating probes for interstitially measuring the temperature, Magnetic resonance imaging (MRI) may be used to measure the temperature while heating the tumour with an interstitially positioned heating probe, such as including a laser source based optical fiber.

The measured temperatures from the provided sensor members, such as the illustrated thermal sensor members 73, 74, 75, are used as input to the control unit 71 for adjusting the power output of the laser generator 72 by adjusting the power of a laser source thereof, for example by a feedback system.

If the temperature at any of the measure points exceeds a predetermined temperature value, the control unit 71 may decrease the power output of the laser generator 72. Alternatively, the control unit 71 may inactivate the laser generator 72.

If the temperature at any of the measure points becomes less than a predetermined temperature value, the control unit 71 may increase the power output of the laser generator 72. Alternatively, the control unit 71 may activate the laser generator 72, if it previously has been inactivated.

The predetermined temperature may be a single maximum or target value. Alternatively, the predetermined temperature may be a range having an upper and a lower threshold temperature.

The adjustment of the output power may be done automatically by the control unit 71. This could for example be done by a control algorithm implemented in the software of the control unit 71. Alternatively and/or additionally, the control unit 71 may provide an alarm, such as to alert a medical practitioner to manually set and/or adjust the power output of the laser generator 52.

The wavelength may be any wavelength as long as there is a suitable absorption in the irradiated tissue to generate heat. Preferably, the wavelength should have a high penetration close to the heating probe, i.e. low absorption. A too high absorption of energy may increase the delivered heat and temperature very rapidly around the heating probe, thus it may not provide enough energy at the boundary of the at least portion of a treatment site covered by the treatment lesion. For example, a wavelength with suitable absorption may be found in the visual or near infrared wavelength region, such as in the region of 700 to 1300nm, such as 900 to 1100nm such as 1064 nm. Also, as known to the skilled person, scattering of the light will have a role in how the energy is transferred within the tissue.

Monitoring the temperature either at the boundary of the at least portion of a tumour or at a distance from the boundary of the treatment lesion may be done by a thermal sensor member 73. The thermal sensor member 20, e.g. a master probe, may be positioned 2-7 mm, such as between 2 to 5 mm, outside an established boundary of a treatment lesion. The choice of distance is depending on the characteristics of the treatment site and the surrounding tissue. The target temperature at the thermal sensor member 73 may be in the range of 40 to 60°C, such as, such as 40 to 55°C, such as 44 to 48°C for obtaining an optimized treatment.

In some examples, the target temperatures may be kept stable for a treatment time between 20 to 60 minutes. Preferably the treatment time may be about 30 minutes. Before starting the treatment time, the target temperatures need to be reached. During this warm-up stage the laser output is adjusted until the right temperature is obtained either at the boundary of the at least portion of a tumour or at the thermal sensor probe 73. The time to target may take between 5 to 15 minutes, such as between 5 to 10 min, depending on the same parameters as previously mentioned, for example, maximum power output and optical coefficients of the different tissues and between different patients.

A heating probe 76 having an energy emitting area is arrangeable in the sleeve. The heating probe 76 may comprise a fibre having a light emitting area at the distal end, wherein the distal end is configured to be interstitially arranged in the tumour. In some examples, the heating probe 10 has capillary arranged over the energy emitting area, such as the light emitting area. Wherein the capped end of the fibre is the distal end, and wherein the distal end is configured to be interstitially arranged in the tissue, such as the tumour. The capillary may, especially when a fibre is used as a heat probe, improve the heat and mechanical stability of the heat probe. In a further example, the light probe is only a fibre having a light emitting area at the distal end, wherein the distal end is configured to be interstitially arranged in the tumour.

When in use, the sleeve may be movably slid along the heating probe 76 in a distal and/or proximal direction to allow positioning of the temperature measuring element 73 at the right distance in relation to an energy emitting area of the heating probe 76 for controlling the thermotherapy by controlling the size of the lesion. The sleeve may be a sleeve arranged around the heating probe 76. The heating probe 76 and the sleeve may be positioned inside an introduced when performing the thermotherapy. Alternatively, the sleeve may be the introduced and the heating probe 76 is arranged inside the introducer when performing the thermotherapy.

Additionally, in some examples of the system the sleeve may have a plurality of temperature measuring elements, 73, 74, 75 arranged in the sleeve. The plurality of temperature measuring elements, 73, 74, 75 may be arranged spaced apart along the sleeve. When using a plurality of temperature measuring elements, 73, 74, 75, the number of temperature measuring elements may be any number larger than 100, such as 2 to 20, such as 2 to 15, such as 2 to 10, such as 2 to 5.

Alternatively, in some examples, at least temperature measuring elements, 73, 74, 75 may be arranged at the same position instead of being spaced apart. This may be done for redundancy to be able to measure the temperature using at least two temperature measuring elements, 73, 74, 75 arranged at the same distance from the emitting area, such as a light emitting area. Should one of the temperature measuring elements, 73, 74, 75 give a false value or no value, the other temperature measuring elements, 73, 74, 75 may be used instead of the one being broken or damaged.

Alternatively, in some examples the temperature measuring elements 73, 74, 75 may be arranged in the heating probe 76 instead of the sleeve. In some examples, temperature measuring elements, 73, 74, 75 may be arranged in both the sleeve and the heating probe 76. Additionally, in some examples, the system 100 may include further external temperature measuring points. These external temperature measuring points may be positioned, for example, next to sensitive anatomical structures that need to be protected from high temperature. These external temperature measuring points may therefore serve as guards shutting off the heat source at a predetermined level to avoid damage to the sensitive structure.

The control unit 70 further comprises a power controlling unit 72, such as an energy source, for controlling the energy emitted by the heat probe 76. The energy causing the heating of the tissue may be emitted using for example RF technology or laser technology. Laser technology may be preferred as it has been shown to improve the control and heating of the tissue to be treated thereby improving the accuracy of optimizing the treatment lesion.

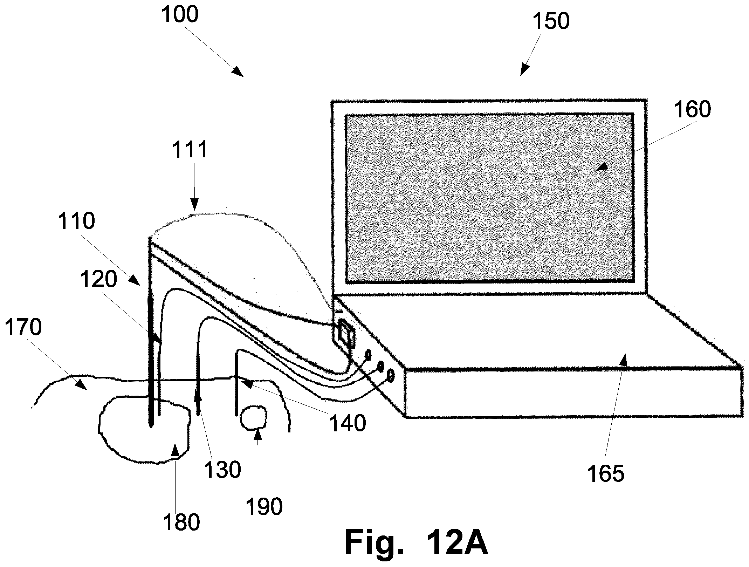

Fig 12A is illustrating an example of a setup of an apparatus 100 for treating a treatment site in tissue by thermotherapy. The main unit 150 comprises a display 160, an input unit 165, such as a keyboard or a mouse. Alternatively and/or additionally, the display 160 could also be an input unit, such as a touch screen. The main unit 150 further comprises a control unit, not illustrated, and a light generator, not illustrated. To the main unit 150 are the heating probe 110 connected via an optical fiber 111. The optical fiber 111 may be connected to the light generator in the main unit 150.

The heating probe 110 is interstitially insertable into a tumour 180 located in, for example a body organ 170. In the illustrated example, the treatment lesion has the same size as the tumour.

In some examples may a first thermal sensor member 130 be used for measuring a temperature in the surrounding tissue outside a boundary of the treatment lesion. The measured temperature may be used in a feedback to the control unit 71 for adjusting the power of the laser. The measured temperature may be used in a feedback to the control unit 71 for adjusting the power of the laser for maintaining the right temperature during the treatment and thereby obtaining the immune response against the treated tumour.

Additionally and/or alternatively, in some organs may a sensitive area 190 that should not be exposed to heat be present in or at a proximity to the tissue being heated. To protect this sensitive area 190, a guard sensor member 140 may be positioned close to the sensitive area 190. The measured temperature at the guard sensor member 140 may be used in a feedback to the control unit for adjusting the power of the laser, hence lowering the temperature at the sensitive area 190.

Additionally and/or alternatively, adjacent the heating probe 110 may a further thermal sensor member 120 positioned. The measured temperature by the further thermal sensor member 120 may be used in a feedback to the control unit 71 for adjusting the power of the laser.

To aid the practitioner with positioning the heat probe 110 and the first thermal sensor member 130 and/or the further thermal sensor member 120 a template may be used. The template may be provided after the treatment site has been investigated using image guidance, e.g. ultrasound.

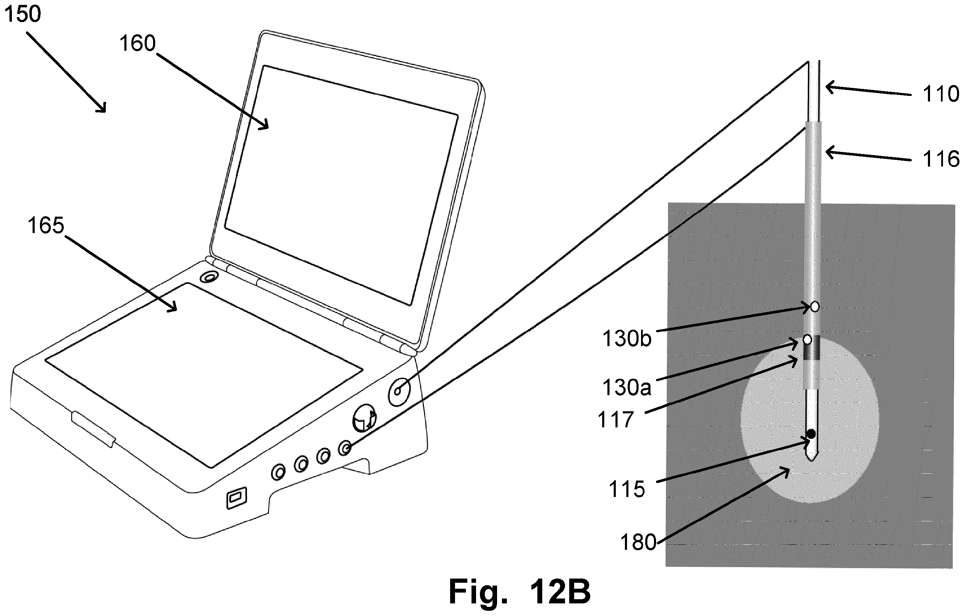

Fig. 12B illustrates a schematic drawing of a system 100 for thermotherapy of tissue. The system comprises a main unit 150. The main unit 150may for example be a computer connected to power control unit to be connected to a heating probe 110. The heating probe includes an optical fibre being connectable to a laser unit. The main unit may also include a temperature reading unit to be connected to temperature measuring elements 130a, 130b. The main unit 150may further include a display unit 160 for displaying information to a practitioner. The information could be, for example, current measured temperature; time lapsed of the treatment; graphs showing changes in the measured temperature over time; size of the lesion; and the power to the heating probe 110. The main unit 150 further includes an input unit 1650, such as a keyboard, a computer mouse, a touch pad, or a touch screen. The main unit 150may further have a first port for connecting the heating probe 110 to the control unit. The control unit may also have a second port for connecting at least one temperature measuring element 120a, 120b, to the main unit 150.

In the system illustrated in Fig. 12B, a heating probe 110 is connected to a main unit 150 via the port. The heating probe includes an optical fibre. The heating probe 110 is connectable to an energy source (not shown), such as a laser source controlled by a power control unit (not shown), such as a laser driver, of the main unit 150.The heating probe 110 has an emitting area at the tip 115. The emitting area may be a light emitting area. The emitting area is configured to be arranged interstitially in the tissue to be treated using the sleeve 116, such as an introducer. When applying energy for heating the tissue, a treatment lesion 180 is obtained covering at least a portion of the tissue to be treated.

In the illustrated examples of the heating probe 110 being an optical fibre, and the emitting area is a light emitting area. The light emitting area may be a bare fibre end or a diffuser. In some examples, the diffuser is a radial fibre. In some other examples, the diffuser is a structured writing in a core and/or cladding and/or buffer of the optical fibre.

Additionally, in some examples the emitting area may be covered with a capillary to improve the heat and mechanical stability during the treatment.

In the schematic illustration, the heating probe 110 is movably arranged as it may slide in an introducer 116. In the introducer is at least one temperature measuring element, 130a, 130b arranged in accordance with the examples given for Fig. 11. In the schematic illustrated figure two temperature measuring element, 130a, 130b are arranged spaced apart along the sleeve. Additionally and/or alternatively, in some examples of the introducer 116, at least two temperature measuring elements are arranged at least at position 130a so that the at least two temperature measuring elements are arranged in the same transverse plane of the sleeve. This may be done for redundancy to be able to measure the temperature using at least two temperature measuring elements arranged at the same distance from the emitting area, such as a light emitting area. Should one of the temperature measuring elements arranged in the same transverse plane of the sleeve give a false value or no value, the other temperature measuring elements may be used instead of the one being broken or damaged.

After the introducer 116 and the heating probe 110 have been arranged in the treatment site, the size of the treating lesion may be determined by sliding the introducer to increase or decrease the distance between the emitting area and the at least one temperature measuring element 130a, 130b. The at least one temperature measuring elements 130a, 130b is used for measuring the temperature which is used to control the heating of the tissue by increasing or decreasing the power to the heating probe, such as adjusting the power to the laser unit connected to the main unit 150.

Additionally and/or alternatively, in some examples when at least two measuring points 130a, 130b are used, if a first measuring point 130a measures a to high temperature after adjustment by sliding the introducer 11, a second measuring point 130b having a longer distance to the emitting area may be used for controlling the treatment.

Additionally and/or alternatively, in some examples when at least two measuring points 130a, 130b are used, if a first measuring point 130a measures and a to high temperature and a second measuring point 130b measures a to low temperature after adjustment by sliding the introducer 116 a virtual point located between the first and the second measurements point 130a, 130b may be used for controlling the treatment. The temperature of the virtual point may be calculated using the measured temperature at the first measuring point 130a and at the second measuring point 130b.

The introducer 116 may in some examples have markers 117 arranged along its length to make it easier to positioning the introducer 116 at the right location by make it visible using ultrasound, MRI, x-ray or other imaging equipment.

As previously described in relation to Fig 11, the system illustrated in Fig. 12B may be combined with a Magnetic Resonance Imaging system which may be used for obtaining a 2D or 3D temperature maps of the treatment area which may be used from defining a treatment lesion. If the system is combined with a Magnetic Resonance Imaging system, the sleeve may not include temperature measuring elements as the temperature at the boarder of the treatment lesion 180 may be measured by the Magnetic Resonance Imaging system.

The foregoing description, for purposes of explanation, used specific nomenclature to provide a thorough understanding of the disclosure. However, it will be apparent to one skilled in the art that the specific details are not required in order to practice the systems and methods described herein. The foregoing descriptions of specific examples are presented for purposes of illustration and description. They are not intended to be exhaustive of or to limit this disclosure to the precise forms described. Obviously, many modifications and variations are possible in view of the above teachings. The examples are shown and described in order to best explain the principles of this disclosure and practical applications, to thereby enable others skilled in the art to best utilize this disclosure and various examples with various modifications as are suited to the particular use contemplated. It is intended that the scope of this disclosure be defined by the claims and their equivalents below.

## Claims

1. A heating probe for performing thermotherapy on at least a portion of a tissue site, comprising:
an optical waveguide which comprising a light emitting area arranged at a distal portion, wherein the distal portion is partially a diffusor made from a structured writing;
the structured writing comprising micro-modifications and the micro-modifications are burnt into a core and/or cladding and/or buffer of said optical waveguide;
wherein the distal portion has an optical axis and the micro-modifications are arranged along the optical axis so that light is emitted from both a lateral surface and an end surface.

2. The heating probe according to claim 1, wherein the micro-modifications are arranged as a helix or spiral along the optical axis.

3. The heating probe according to claim 2, wherein the micro-modifications are arranged as at least two helices or spirals, wherein each spiral and helix has a different radii.

4. The heating probe according to claim 3, wherein the helices and spirals are arranged with an offset in relation to each other in a direction of the optical axis.

5. The heating probe according to any of claims 2 and 3, wherein the number of helices and spirals varies along the optical axis.

6. The heating probe according to claim 2, wherein the micro-modifications are arranged as a plurality of lines arranged along the optical axis.

7. The heating probe according to claim 5, wherein the lines has different distances from the optical axis.

8. The heating probe according to any of claims 6 and 7, wherein the number of lines varies along the optical axis.

9. The heating probe according to any of claims 1 to 8, wherein the density of the micro-modifications varies along the optical axis to control the portion of light being emitted from the lateral surface and the end surface.

10. The heating probe according to claim 9, wherein the density increase towards the end surface.

11. The heating probe according to any of claims 9 and 10, wherein the density vary by varying the distance between the micro-modifications along the optical axis.

12. The heating probe according to any of claims 1 to 11, further comprising a capillary arranged to cover at least said light emitting area to protect the emitting area.

13. The heating probe according to claim 12, wherein the capillary is configured to lower energy density in an interface between the heating probe and the tissue.

14. The heating probe according to any of claims 1 to 13, wherein tissue is
brain tissue for treatment of a neurological disease, such as brain tumour, radiation necrosis or epilepsy.

15. A system for performing thermotherapy on at least a portion of a tissue site, comprising:
a light source configured to emit light;
a heating probe configured to be inserted into the tissue site,
comprising:
an optical waveguide which comprising a light emitting area arranged at a distal portion, wherein the distal portion is partially a diffusor made from a structured writing;
the structured writing comprising micro-modifications and the micro-modifications are burnt into a core and/or cladding and/or buffer of said optical waveguide; and
wherein the distal portion has an optical axis and the micro-modifications are arranged along the optical axis so that light are emitted from both a lateral surface and an end surface.

16. The system according to claim 15, wherein magnetic resonance imaging (MRI) is used for monitoring the heat distribution in the tissue site and/or tissue surrounding the tissue site, or at least one temperature probe.
